(19) Europäisches Patentamt
European Patent Office
Office européen des brevets

(11) Numéro de publication: **0 401 093 B1**

# (12) FASCICULE DE BREVET EUROPEEN

(45) Date de publication de fascicule du brevet: **01.03.95**

(51) Int. Cl.$^6$: **C07D 207/48**, C07D 401/12, C07D 403/12, C07D 405/12, C07D 401/14, C07D 403/06, C07D 417/06, A61K 31/40

(21) Numéro de dépôt: **90401387.7**

(22) Date de dépôt: **23.05.90**

Le dossier contient des informations techniques présentées postérieurement au dépôt de la demande et ne figurant pas dans le présent fascicule.

## (54) Nouveaux dérivés de la pyrrolidone, leur procédé de preparation et les compositions pharmaceutiques les renfermant.

(30) Priorité: **31.05.89 FR 8907153**

(43) Date de publication de la demande:
**05.12.90 Bulletin 90/49**

(45) Mention de la délivrance du brevet:
**01.03.95 Bulletin 95/09**

(84) Etats contractants désignés:
**AT BE CH DE DK ES FR GB GR IT LI LU NL SE**

(56) Documents cités:
**EP-A- 0 138 721**
**EP-A- 0 229 566**

(73) Titulaire: **ADIR ET COMPAGNIE**
**1 rue Carle Hébert**
**F-92415 Courbevoie Cédex (FR)**

(72) Inventeur: **Regnier, Gilbert**
**27 Av. du Plessis**
**F-92290 Chatenay Malabry (FR)**
Inventeur: **Dhainaut, Alain**
**7 rue des Guipières**
**F-78400 Chatou (FR)**
Inventeur: **Lepagnol, Jean**
**5 rue de Vlaminck**
**F-78400 Chatou (FR)**

(74) Mandataire: **Reverbori, Marcelle**
**ADIR**
**1, rue Carle Hébert**
**F-92415 Courbevoie Cédex (FR)**

EP 0 401 093 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des nouveaux dérivés de la pyrrolidone, leur procédé de préparation et les compositions pharmaceutiques les renfermant.

Elle concerne particulièrement les dérivés de la pyrrolidone de formule générale I :

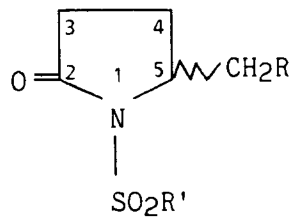

(I)

dans laquelle :

- R représente un groupe

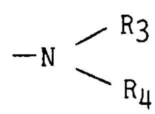

dans lequel :

. $R_3$ et $R_4$, identiques ou différents, représentent chacun :
un atome d'hydrogène,
un radical alkyle ayant jusqu'à 6 atomes de carbone, en chaîne droite ou ramifiée ou

. $R_3$ et $R_4$ forment ensemble avec l'atome d'azote auquel ils sont liés un radical hétérocyclique de formule

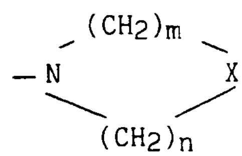

dans laquelle :

- m représente zéro, un ou deux ;
- n est un nombre entier de 1 à 3, et
- X représente :
    - un radical méthylène - $CH_2$ - ;
    - un atome d'oxygène ou de soufre, ou le radical $SO_2$ ;
    - un radical

      $>N-R_5$

      dans lequel $R_5$ représente :

      . un radical alkyle ayant de 1 à 4 atomes de carbone,

      . un radical hydroxyalkyle ou aralkyle dans lequel la partie aryle est éventuellement substituée par un ou plusieurs radicaux alkoxy (ayant, de 1 à 4 atomes de carbone) ou méthylènedioxy, et où, dans chacun d'entre eux, la partie alkyle renferme de 1 à 4 atomes de carbone,

      . un radical aryle éventuellement substitué par un ou plusieurs atomes d'halogène ou radicaux alkoxy ayant de 1 à 4 atomes de carbone, ou

      . un radical hétérocyclique, ayant un ou deux hétéroatomes, tel que, par exemple, un radical thiazolyle, pyrimidinyle, pyrazinyle, pyridyle ou quinolyle chacun éventuellement substitué par un ou plusieurs atomes d'halogène ou radicaux trifluorométhyle ou alkoxy

ayant de 1 à 4 atomes de carbone ; ou,

- un radical

$>$CH - YZ

dans lequel Y représente un atome d'oxygène ou de soufre et Z représente un radical hétérocyclique, ayant 1 ou 2 hétéroatomes, tel que défini ci-dessus ;

- R' représente :

  a) un radical alkyle ayant de 1 à 4 atomes de carbone en chaîne droite ou ramifiée, comportant éventuellement une double liaison et / ou éventuellement substitué par un ou plusieurs radicaux hydroxy, trifluorométhyle, amino, ou phényle lui-même éventuellement substitué par un ou plusieurs atomes d'halogène ou radicaux alkyle ou alkoxy (ayant chacun de 1 à 5 atomes de carbone), nitro ou amino ; ou

  b) un radical aryle éventuellement substitué par un ou plusieurs atomes d'halogène ou radicaux alkyle ou alkoxy (ayant chacun de 1 à 5 atomes de carbone), nitro ou amino, et,

- le carbone situé en position 5 du cycle pyrrolidone, substitué par $CH_2R$, possède la configuration R ou S selon que la matière première de synthèse utilisée est l'acide pyroglutamique naturel de configuration R ou S.

L'état antérieur de la technique est illustré notamment par la demande de brevet européen publiée sous le n° 0.229.566 qui a pour objet les benzènesulfonyl - 1 oxo - 2 pyrrolidines toujours substituées en position - 5 par une fonction éther, lesquelles sont utilisables comme médicaments dans le traitement des asthénies intellectuelles ou nerveuses, des défaillances de la mémoire, de la sénescence et du surmenage intellectuel.

Les dérivés de la présente invention diffèrent des dérivés de la demande européenne n° 0.229.566 en ce qu'ils ne possèdent jamais de fonction éther directement liée au noyau pyrrolidine. Toutefois, ils possèdent une activité pharmacologique et thérapeutique particulièrement intéressante comme le prouve l'étude pharmacologique décrite dans l'exemple 49.

La présente invention a également pour objet le procédé de préparation des dérivés de formule générale I, caractérisé en ce que l'on fait réagir un dérivé de la pyrrolidone de formule générale II :

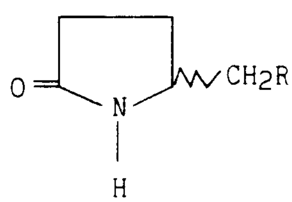

(II)

dans laquelle R a la signification précédemment définie avec un sulfochlorure de formule générale III :

$Cl\ SO_2 - R'$ (III)

dans laquelle R' a la signification précédemment définie.

Il est particulièrement avantageux de faire réagir le dérivé II, préalablement transformé au moyen d'hydrure de sodium ou de butyl lithium respectivement en dérivé sodé ou lithié, avec le sulfochlorure III, à température ambiante, dans un solvant aprotique polaire tel que, par exemple, le tétrahydrofuranne, le dioxane, le diméthylsulfoxide, le diméthylformamide ou leur mélange.

La présente invention a également pour objet une variante du procédé ci-dessus,caractérisé en ce que l'on fait réagir un dérivé de la pyrrolidone de formule générale IV :

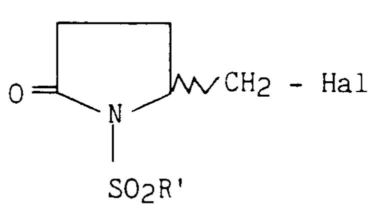

(IV)

dans laquelle R' a la signification précédemment définie et Hal représente un atome de chlore ou de brome,

avec une amine secondaire de formule générale V:

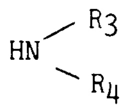

(V)

dans laquelle $R_3$ et $R_4$ ont les significations précédemment définies. La réaction s'effectue de façon particulièrement adéquate en chauffant les dérivés (IV) et (V) dans un solvant polaire tel que, par exemple, un alcool à haut point d'ébullition, le diméthylformamide ou le diméthylacétamide, à une température comprise entre 110 et 150 °C, en présence d'un accepteur de l'hydracide formé au cours de la réaction Cet accepteur peut être une base tertiaire telle que, par exemple, la triéthylamine ou l'éthyl dipropylamine, ou un excès de l'amine secondaire (V), laquelle peut également, éventuellement servir de solvant réactionnel.

Les matières premières de formule II peuvent être préparées par chauffage d'un dérivé halogéné de formule X :

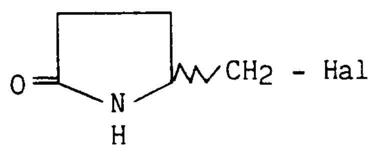

(X)

dans laquelle Hal représente un atome de chlore ou de brome, [lui-même préparé selon G. WILEY et Coll. J. Am. Chem. Soc 86, 964 (1964), au moyen de $P(C_6H_5)_3$-$CCl_4$ ou $P(C_6H_5)_3$ $Br_2$ dans le diméthylformamide], avec une amine de formule :

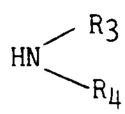

dans laquelle $R_3$ et $R_4$ ont les significations précédemment définies

Les matières premières de formule IV peuvent être aisément préparées par halogénation des dérivés de formule XI :

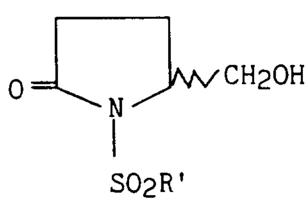

(XI)

dans laquelle R' a la signification précédemment définie, eux-même préparés par hydrolyse ménagée des dérivés de formule XII :

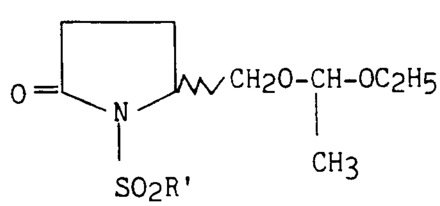

(XII)

dans laquelle R' a la signification précédemment définie, lesquels, dérivés XII étant eux-mêmes préparés à partir d'un halogénure de sulfonyle III et du dérivé IX.

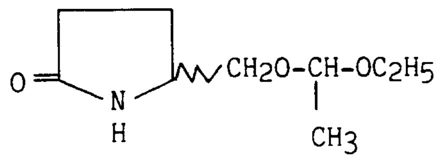

(IX)

Tous ces composés peuvent être purifiés par des méthodes chimiques classiques telles que distillation, cristallisation à l'état de sels ou bien par chromatographie flash sur support de silice (35 - 70 μ) avec des systèmes d'élution tels que $CH_2Cl_2$ - méthanol, $CH_3COOC_2H_5$ - méthanol, $CH_3COOC_2H_5$, etc..., sous des pressions d'azote comprises entre 0,5 et 1 bar.

Les dérivés de l'invention peuvent être transformés en sel d'addition avec les acides, sels, qui font, à ce titre, partie de la présente invention. Comme acides utilisables pour la formation de ces sels, on peut citer par exemple dans la série minérale : les acides chlorhydrique, bromhydrique, sulfurique, phosphorique et dans la série organique : les acides acétique, propionique, maléique, fumarique, tartrique, citrique, benzoïque et méthane sulfonique.

Les dérivés de formule générale I et leurs sels physiologiquement tolérables possèdent des propriétés pharmacologiques et thérapeutiques intéressantes.

Notamment, ils s'opposent aux désordres cérébraux liés à une défaillance énergétique par défaut d'apport d'oxygène, ou liés à une insuffisance métabolique neuronale. Ils peuvent de ce fait être utilisés dans le traitement des asthénies, des syndromes ischémiques aigus, transitoires ou progressifs et des maladies nerveuses liées au vieillissement normal ou pathologique tel que la maladie d'Alzheimer. Le dosage unitaire peut être de 1 à 1000 mg pour un traitement en 1 à 3 prises par jour.

La présente invention a également pour objet les compositions pharmaceutiques contenant comme principe actif un composé de formule générale I ou un de ses sels physiologiquement tolérables, mélangé ou associé a un excipient pharmaceutique approprié comme par exemple l'eau distillée, le glucose, le lactose, l'amidon, le talc, l'éthylcellulose, le stéarate de magnésium ou le beurre de cacao. Les compositions pharmaceutiques ainsi obtenues se présentent généralement sous forme dosée et peuvent contenir de 1 à 1000 mg de principe actif. Elles peuvent revêtir la forme de comprimés, dragées, gélules, solutions buvables, solutions injectables ou suppositoires et être, selon les cas, administrées à la dose de 1 à 1000 mg 1 à 3 fois par jour.

Les exemples suivants illustrent l'invention. Les points de fusion sont déterminés au tube capillaire.

**EXEMPLE 1**

**S - BENZENESULFONYL - 1 OXO - 2 MORPHOLINOMETHYL - 5 PYRROLIDINE :**

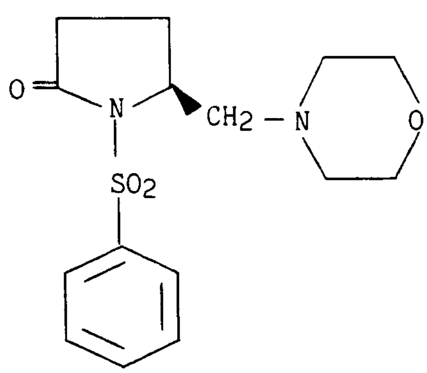

A) 1ère méthode :

A une solution de 1,85 g de S -morpholinométhyl - 5 pyrrolidone - 2 (fondant à 74 - 77 °C) dans 20 ml de tétrahydrofuranne anhydre, on ajoute à 10 °C, sous azote, 7 ml de Bu Li (1,6 M) en solution dans

l'hexane. On agite 30 minutes à température ambiante, puis on ajoute 1,8g de chlorure de benzènesulfonyle en maintenant la température à 15 °C. On maintient l'agitation du mélange pendant 15 heures à température du laboratoire puis on évapore le solvant sous pression réduite. On reprend le résidu par 20 ml d'eau et 20 ml de $CH_2Cl_2$. On décante et sèche sur Mg $SO_4$ et purifie le résidu d'évaporation par chromatographie flash sur $SiO_2$ avec $CH_3COOC_2H_5$ 100 % comme éluant.

On isole ainsi 1,6 g de cristaux de S - benzènesulfonyl - 1 oxo - 2 morpholinométhyl - 5 pyrrolidine. Le S - morpholinométhyl - 5 pyrrolidone - 2 de départ

$$[\alpha_D^{20,5} = + 48,3\ ° \ (C = 1\ C_2H_5OH)]$$

a été préparé avec un rendement de 78 % par chauffage à reflux du S - chlorométhyl - 5 pyrrolidone - 2 (P.F. : 54 - 56 °C) dans la morpholine ; le composé chlorométhylé étant lui-même préparé, par la méthode $(C_6H_5)_3P$ - $CCl_4$ avec un rendement de 86 %, à partir du S - pyroglutaminol.

De la même façon, en utilisant du R - morpholinométhyl - 5 pyrrolidone -2 ( à la place du S - morpholinométhyl - 5 pyrrolidone - 2), on obtient la R - benzènesulfonyl - 1 oxo - 2 morpholinométhyl - 5 pyrrolidine.

B) <u>2ème méthode</u> :

On chauffe pendant 3 heures à reflux une solution de 2,7 g de benzènesulfonyl - 1 S - chlorométhyl - 5 pyrrolidone - 2 dans la morpholine. On chasse ensuite l'excès de morpholine sous pression réduite et reprend le résidu avec $H_2O$. On essore les cristaux et les sèche sous vide. On obtient 3,1 g de cristaux de S - benzènesulfonyl - 1 oxo - 2 morpholinométhyl - 5 pyrrolidine fondant à 113 - 114 °C. Le dérivé chlorométhylé de départ (huile) a été préparé par la méthode $P(C_6H_5)_3$ -$CCl_4$ - diméthylformamide, avec un rendement de 85 %, à partir du benzènesulfonyl - 1 S - pyrroglutaminol, P.F. : 104 - 105 °C, $\alpha_D^{21}$ : - 51,2 ° (C = 1 $CH_3OH$).

**<u>EXEMPLE 2</u>** :

**S - BENZENESULFONYL - 1 OXO - 2 [(METHYL - 4 PIPERAZINYL - 1)METHYL] - 5 PYRROLIDINE,**

dont les constantes physiques du dichlorhydrate sont : P.F. : 230 °C; $\alpha_D^{21}$ = - 42,4 ° (C = 1 % $CH_3OH$) préparée comme décrit dans l'exemple 1 première méthode à partir de S - [(méthyl - 4 pipérazinyl - 1) méthyl] - 5 pyrrolidone - 2 (huile).

**<u>EXEMPLE 3</u>** :

**S - BENZENESULFONYL - 1 OXO - 2 {[(TRIMETHOXY - 2, 3, 4 BENZYL) - 4 PIPERAZINYL - 1] METHYL} - 5 PYRROLIDINE,**

dont les constantes du dichlorhydrate sont : P.F. : 235 - 238 °C, $\alpha_D^{20}$ = - 21,7 ° (C = 1 %, $H_2O$) préparée comme décrit dans l'exemple 1 première méthode à partir de S -{[(trimethoxy - 2, 3, 4 benzyl) - 4 piperazinyl - 1[ methyl} - 5 pyrrolidone - 2 (huile).

**<u>EXEMPLE 4</u>** :

**S - BENZENESULFONYL - 1 OXO - 2 [(METATRIFLUOROMETHYLPHENYL - 4 PIPERAZINYL - 1) METHYL] - 5 PYRROLIDINE,**

dont les constantes physiques du fumarate sont : P.F. : 151 °C ; $\alpha_D^{20}$ = - 36,9 ° (C = 1 %, $C_2H_5OH$), préparée comme décrit dans l'exemple 1 première méthode à partir de S - [(metatrifluoromethylphenyl - 4 piperazinyl - 1) methyl] - 5 pyrrolidone - 2.

**EXEMPLES 5 - 48** :

En opérant comme dans l'exemple 1 première méthode ont été préparés les dérivés regroupés dans le tableau ci-après :

Dérivés de la pyrrolidone de formule :

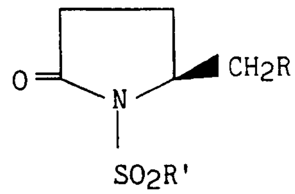

| EXEMPLE | R | R' |
|---|---|---|
| 5 | 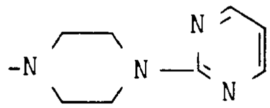 | 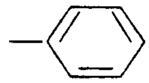 |
| 6 | 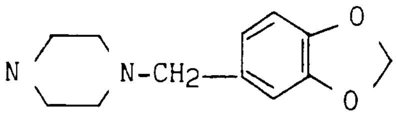 | " |
| 7 | 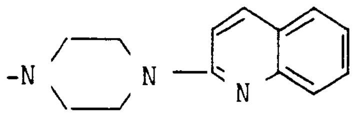 | " |
| 8 | 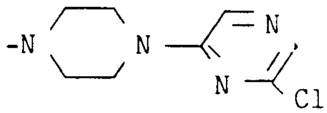 | " |
| 9 | 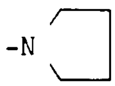 | " |
| 10 | 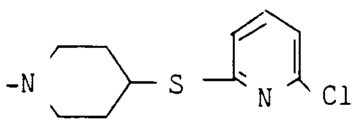 | " |
| 11 | 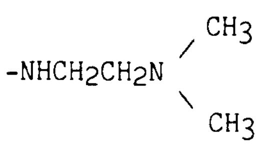 | " |
| 12 | 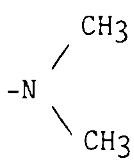 | " |
| 13 | 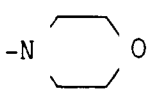 | 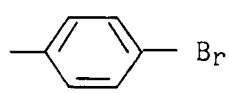 |

| EXEMPLE | R | R' |
|---|---|---|
| 14 | 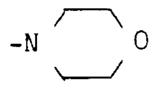 | 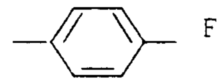 |
| 15 | " | 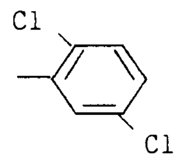 |
| 16 | " | 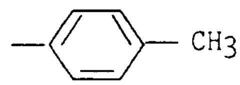 |
| 17 | " | 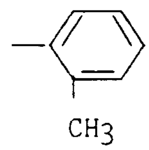 |
| 18 | " | 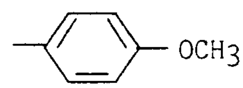 |
| 19 | " | 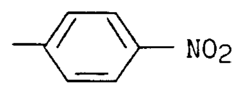 |
| 20 | " | 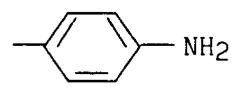 |
| 21 | " | $-CH_3$ |
| 22 | " | $-CH_2-CH_3$ |
| 23 | " | 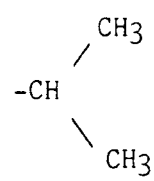 |
| 24 | " | $-(CH_2)_3-CH_3$ |
| 25 | " | $-(CH_2)_6-CH_3$ |
| 26 | " | $-(CH_2)_9-CH_3$ |

| EXEMPLE | R | R' |
|---|---|---|
| 27 | 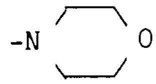 | $-CH_2-CF_3$ |
| 28 | " | $-CH_2-CH=CH_2$ |
| 29 | " | 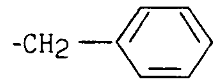 |
| 30 | " | 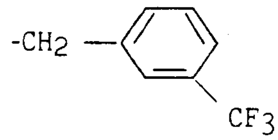 |
| 31 | " | 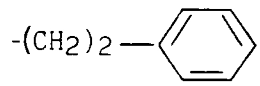 |
| 32 | 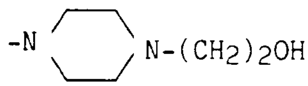 | 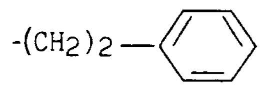 |
| 33 | 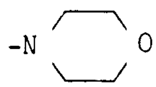 | 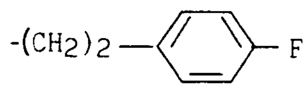 |
| 34 | 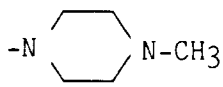 | 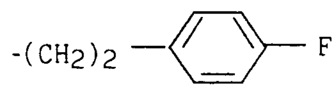 |
| 35 | 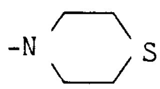 | 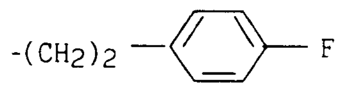 |
| 36 | 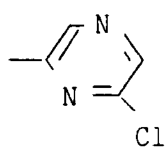 | 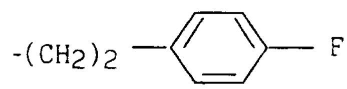 |
| 37 | 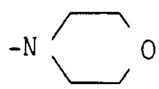 | 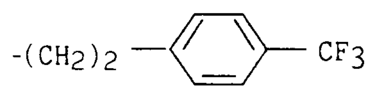 |
| 38 | 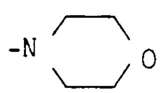 | 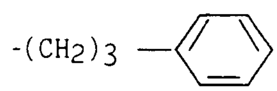 |
| 39 | 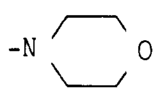 | 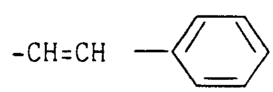 |

| EXEMPLE | R | R' |
|---|---|---|
| 40 | 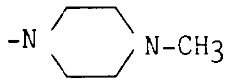 | 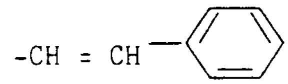 |
| 41 | 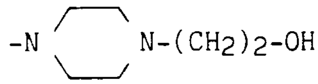 | 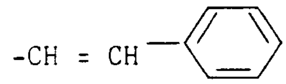 |
| 42 | 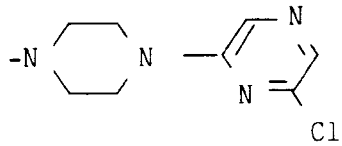 | 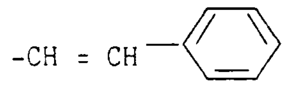 |
| 43 | 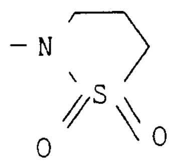 | 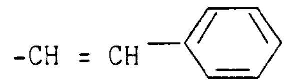 |
| 44 | 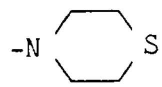 | 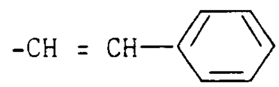 |
| 45 | 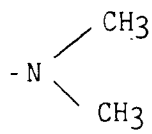 | 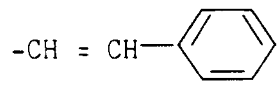 |
| 46 | 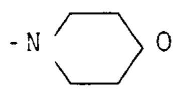 | 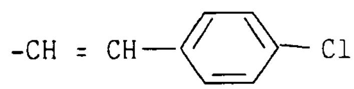 |
| 47 | 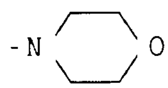 | $-CH_2-CH_2-NH_2$ |
| 48 | 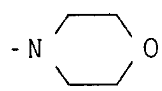 | 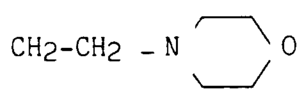 |

En opérant comme décrit dans l'exemple 1 première méthode, à partir de matière sous forme R, on obtient tous les dérivés objet des exemples 1 à 48 sous forme R, lesquels ne diffèrent des dérivés S correspondant, en ce qui concerne leurs caractéristiques physiques, que par leur pouvoir rotatoire.

**EXEMPLE 49** :

**ETUDE PHARMACOLOGIOUE**

a) Principe

Lors du vieillissement, la baisse des réserves intrinsèques cérébrales se traduit par l'incapacité à répondre à toute nouvelle demande métabolique. Lors d'un accident vasculaire cérébral, la vulnérabilité sélective neuronale apparaît dans les cellules qui ont la plus forte demande métabolique. Le point commun entre ces pathologies est le déficit d'utilisation qui mène le plus souvent à la déviation du métabolisme vers l'anaérobie.

De telles agressions peuvent être reproduites expérimentalement et de façon aigüe, par l'hypoxie pour la recherche de nouveaux composés cérébroprotecteurs.

En effet, l'analogie entre l'hypoxie et les désordres cérébraux liés aux vieillissement ou à l'insuffisance circulatoire, s'expriment notamment par :

- La baisse des réserves énergétiques
- la moindre résistance au stress
- la baisse de la synthèse oxygéno-dépendante des neurotransmetteurs
- la chute des capacités mnésiques.

Les composés de la présente invention ont donc été testés sur leurs capacités à prolonger la survie du tissu cérébral lors d'hypoxie aigüe chez la Souris.

Leur efficacité a été comparée à celle des substances de référence reconnues comme agents antihypoxiques ou comme agents nootropes.

D'autre part, leurs effets antihypoxiques cérébroprotecteurs ont été comparés aux éventuels effets cérébrovasculaires grâce à l'utilisation de deux tests complémentaires : l'arrêt cardiaque aigu et l'anoxie aigüe à l'azote chez la Souris.

Cette comparaison permet en effet de discriminer les composés antihypoxiques "purs", protecteurs neuronaux, qui exercent un tropisme cérébral prépondérant lors d'hypoxie, des composés non spécifiques qui manifestent également leurs effets protecteurs lors d'ischémie cardiaque et/ou lors d'anoxie généralisée. Dans ce dernier cas, l'effet systématique d'un composé est dû, le plus souvent à des effets vasodilatateurs et/ou sédatifs.

**b) Méthologie**

**α) Hypoxie hypobare aigüe**

Des souris CD1 (Charles River) de sexe mâle ayant reçu 30 minutes auparavant par voie IP, le composé étudié, sont soumises à une hypoxie aigüe de type hypobare. Pour cela, elles sont placées dans une enceinte où la pression barométrique peut être abaissée rapidement (30 secondes) à une valeur de 160 mbar.Le renouvellement d'air assuré durant l'épreuve permet de provoquer une hypoxie et non une asphyxie ou une anoxie qui pourraient faire appel à une protection d'origine vasculaire. Dans ces conditions, la mort cérébrale des animaux est obtenue en 15 secondes environ après l'obtention de la pression hypoxique.

Le temps moyen de survie d'un lot traité est comparé à celui d'un lot témoin ne recevant que le solvant.

**β) Ischémie cardiaque aigüe**

Des souris CD1 (Charles River) de sexe mâle ayant reçu 30 minutes auparavant par voie IP, le composé étudié, sont soumises à un arrêt cardiaque brutal par injection intraveineuse d'une dose massive de MgCl2 (0,1 ml d'une solution de sérum physiologique saturé en MgCl2). Dans ces conditions, la mort cérébrale des animaux est obtenue en 20 secondes environ après l'injection IV.

Le temps moyen de survie d'un lot traité est comparé à celui d'un lot témoin ne recevant que le solvant.

**Υ) Anoxie générale aigûe à l'azote pur** Des souris CD1 (Charles River) de sexe mâle ayant reçu 30 minutes auparavant par voie IP, le composé étudié, sont soumises à une anoxie aigüe par inhalation d'azote pur. Pour cela, elles sont placées dans une enceinte de volume réduit (250 ml) où circule sans surpression, à grand débit (2 l/minutes) un courant d'azote pur. Dans ces conditions, la mort cérébrale des animaux est obtenue en 24 secondes environ après déclenchement du courant anoxique.

Le temps moyen de survie d'un lot traité est comparé à celui d'un lot témoin ne recevant que le solvant.

**c) Résultats**

Dans ces conditions expérimentales drastiques, les composés de référence n'exercent qu'un effet antihypoxique modéré ou s'accompagnant d'effets d'intensité équivalente sur l'un ou sur les deux tests complémentaires. Dans ce dernier cas, une vasodilatation apparente et/ou une sédation accompagnent ces effets non spécifiques.

Les composés nootropes de référence : piracetam et analogues, ne protègent que faiblement le cerveau de l'hypoxie. De fortes doses sont parfois nécesaires pour observer une augmentation significative du temps de survie cérébrale.

| | | |
|---|---|---|
| Piracetam | 300 mg/kg<br>1000 mg/kg | +5,3 sec. (34 %)<br>+6,3 sec. (+40%) |
| Pramiracetam | 100 mg/kg<br>300mg/kg | +3,3 sec. (+21%)<br>+5,6 sec. (+36%) |
| Oxiracetam | 100 mg/kg | +1,1 sec. (+6%) |
| Doliracetam | 30 mg/kg<br>100mg/kg | +0,1 sec. (+1%)<br>+15,3 sec. (+107%) |

Dans le cas du doliracetam, cet effet protecteur exercé à la dose de 100mg/kg s'accompagne de sédation indésirable, ce qui le différencie des autres composés nootropes. Cette sédation est d'ailleurs responsable de l'effet antihypoxique.
D'autres composés de référence ont été étudiés. Ils ont été sélectionnés pour leurs indications thérapeutiques de type cérébroprotecteur. Les résultats suivants ont été obtenus :

| | | |
|---|---|---|
| Nizophenone | 3mg/kg | +12,1 sec. (+86%) |
| Pyritinol | 100mg/kg | +3,9 sec. (+27%) |
| Dihydroergotoxine | 10mg/kg | +9,6 sec. (+68%) |
| Meclofenoxate | 100mg/kg | +3,1 sec. (+22%) |

Aux doses étudiées pour obtenir un effet antihypoxique marqué, la nizophenone et la dihydroergotoxine exercent des effets secondaires, à savoir respectivement sédation et vasodilatation périphérique.
Les composés de l'invention qui n'entraînent pas d'effets neurocomportementaux indésirables, s'opposent très significativement à l'hypoxie cérébrale, comme l'indiquent les exemples suivants :

| | | |
|---|---|---|
| **Exemple 8** | 100mg/kg | +25,9 sec (+159%) |
| **Exemple 29** | 100mg/kg | +14,5 sec.(+88%) |
| **Exemple 40** | 100mg/kg | +18,8 sec. (+156%) |
| **Exemple 43** | 100mg/kg | +10,5 sec.(+74%) |
| **Exemple 44** | 100mg/kg | +48,9 sec.(+345%) |

Ces effets antihypoxiques cérébraux, neuroprotecteurs s'exercent avec une grande spécificité comme le montrent les résultats comparés entre les trois tests utilisés, précédemment décrits, qui sont regroupés dans le tableau suivant.
Ces résultats sont exprimés en valeurs absolues d'augmentation du temps de survie (secondes).

| Composé | Dose (mg/kg) | Hypoxie cérébrale | Arrêt cardiaque | Anoxie Générale |
|---|---|---|---|---|
| Piracetam | 1000 | + 6,3 | 0 | + 1,6 |
| Pramiracetam | 100 | + 3,3 | + 0,4 | 0 |
| Doliracetam | 100 | + 15,3 | + 6,4 | + 14,6 |
| Nizophenone | 3 | + 12,1 | +10,5 | + 10,7 |
| Pyritinol | 100 | + 3,9 | + 1,9 | + 5,0 |
| Dihydroergotoxine | 10 | + 9,6 | + 5,3 | + 10,1 |
| Prazosine | 3 | + 3,9 | + 4,4 | + 6,9 |
| Exemple 8 | 100 | + 25,9 | + 4,3 | + 10,6 |
| Exemple 29 | 100 | + 14,5 | + 4,3 | + 7,0 |
| Exemple 40 | 100 | + 18,8 | + 3,4 | + 9,1 |
| Exemple 43 | 100 | + 10,5 | + 0 | + 2,8 |
| Exemple 44 | 100 | + 48,9 | +15,5 | +13,8 |

Les composés de la présente invention exercent donc un effet antihypoxique puissant et sélectif. Ils se différencient des composés pris en référence, soit par l'intensité de leurs efffets (versus piracetam, pramiracetam, pyritinol), soit par leur spécificité grâce à l'absence d'effet sédatif (versus doliracetam,

nizophenone) ou vasculaire (versus dihydroergotoxine, prazosine). En effet, dans ces derniers cas, les composés de référence exercent des effets identiques voire supérieurs vis-à-vis de l'arrêt cardiaque ou de l'anoxie généralisée, ce qui indique une absence de tropisme prépondérant pour le cerveau ou un effet indirect.

Les composés de l'invention peuvent donc s'opposer aux désordres cérébraux liés à une défaillance énergétique notamment par défaut d'apport en oxygène, et à toute insuffisance métabolique neuronale.

**Revendications**

**Revendications pour les Etats contractants suivants : AT, BE, CH, DE, DK, FR, GB, IT, LI, LU, NL, SE,**

**1.** Les dérivés de la pyrrolidone de formule générale I :

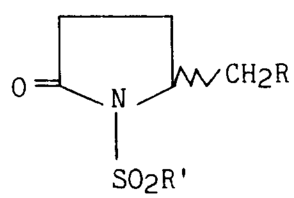

(I)

dans laquelle :

- R représente :
  un groupe

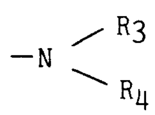

dans lequel :

. $R_3$ et $R_4$, identiques ou différents, représentent chacun :
un atome d'hydrogène,
un radical alkyle ayant jusqu'à 6 atomes de carbone, en chaîne droite ou ramifiée, ou

. $R_3$ et $R_4$ forment ensemble avec l'atome d'azote auquel ils sont liés un radical hétérocyclique de formule

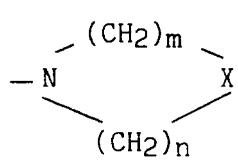

dans laquelle :

- m représente zéro, un ou deux ;
- n est un nombre entier de 1 à 3, et
- X représente :
  - un radical méthylène - $CH_2$ - ;
- un atome d'oxygène ou de soufre, ou le radical $SO_2$ ;
- un radical N-$R_5$ dans lequel $R_5$ représente :
  . un radical alkyle ayant de 1 à 4 atomes de carbone,
  . un radical hydroxyalkyle ou aralkyle dans lequel la partie aryle est éventuellement substituée par un ou plusieurs radicaux alkoxy (ayant de 1 à 4 atomes de carbone) ou méthylènedioxy, et où, dans chacun d'entre eux, la partie alkyle renferme de 1 à 4 atomes de carbone,

. un radical aryle éventuellement substitué par un ou plusieurs atomes d'halogène ou radicaux alkoxy ayant de 1 à 4 atomes de carbone, ou

. un radical hétérocyclique, ayant un ou deux hétéroatomes, choisi parmi les radicaux thiazolyle, pyrimidinyle, pyrazinyle pyridyle et quinolyl, et chacun de ces radicaux substitué par un ou plusieurs atomes d'halogène ou radicaux trifluorométhyle ou alkoxy ayant de 1 à 4 atomes de carbone ; ou,

- un radical

>CH - YZ

dans lequel Y représente un atome d'oxygène ou de soufre et Z représente un radical hétérocyclique, ayant 1 ou 2 hétéroatomes, tel que défini ci-dessus;

- R' représente :
a) un radical alkyle ayant de 1 à 4 atomes de carbone en chaîne droite ou ramifiée, comportant éventuellement une double liaison et / ou éventuellement substitué par un ou plusieurs radicaux hydroxy, trifluorométhyle, amino ou phényle lui-même éventuellement substitué par un ou plusieurs atomes d'halogène ou radicaux alkyle ou alkoxy (ayant chacun de 1 à 5 atomes de carbone), nitro ou amino ; ou
b) un radical aryle éventuellement substitué par un ou plusieurs atomes d'halogène ou radicaux alkyle ou alkoxy (ayant chacun de 1 à 5 atomes de carbone), nitro ou amino,
et,
- le carbone situé en position 5 du cycle pyrrolidone, substitué par $CH_2R$, possède la configuration R ou S.

**2.** Les sels d'addition acides des dérivés de la revendication 1.

**3.** Les sels selon la revendication 2 qui sont physiologiquement tolérables.

**4.** Un composé de la revendication 1 qui est :
la S - benzènesulfonyl - 1 oxo - 2 morpholinométhyl - 5 pyrrolidine.

**5.** Un composé de la revendication 1 qui est :
la S - benzènesulfonyl - 1 oxo - 2 [(méthyl - 4 pipérazinyl - 1) méthyl] - 5 pyrrolidine.

**6.** Un composé de la revendication 1 qui est :
la S - benzènesulfonyl - 1 oxo - 2 [(métatrifluorométhylphényl - 4 pipérazinyl - 1) méthyl] - 5 pyrrolidine.

**7.** Un composé de la revendication 1 qui est :
la S-méthanesulfonyl-1 oxo-2 morpholinométhyl-5 pyrrolidine.

**8.** Un composé de la revendication 1 qui est :
la S - benzènesulfonyl - 1 oxo - 2 {[(chloro - 6 pyrazinyl - 2) - 4 pipérazinyl - 1] méthyl} - 5 pyrrolidine, et son chlorhydrate.

**9.** Un composé de la revendication 1 qui est :
la S - benzylsulfonyl - 1 oxo - 2 morpholinométhyl - 5 pyrrolidine et son chlorhydrate.

**10.** Un composé de la revendication 1 qui est :
la S - styrylsulfonyl - 1 oxo - 2 [(méthyl - 4 pipérazinyl - 1) méthyl] - 5 pyrrolidine.

**11.** Un composé de la revendication 1 qui est :
la S-styrylsulfonyl - 1 oxo - 2 thiomorpholinométhyl - 5 pyrrolidine.

**12.** Le procédé de préparation des dérivés de la revendication 1 caractérisé en ce que l'on fait réagir un dérivé de la pyrrolidone de formule générale II :

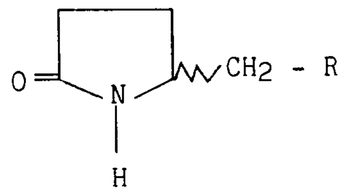

(II)

dans laquelle R a la signification définie dans la revendication 1, avec un sulfochlorure de formule générale III :

$Cl\ SO_2 - R'$ (III)

dans laquelle R' a la signification définie dans la revendication 1.

**13.** Le procédé de préparation des dérivés de la revendication 1, caractérisé en ce que l'on fait réagir un dérivé de la pyrrolidone de formule générale IV :

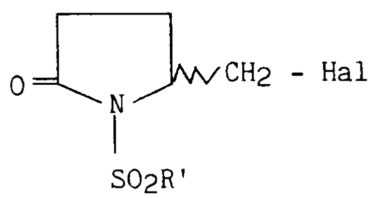

(IV)

dans laquelle R' a la signification définie dans la revendication 1 et Hal représente un atome de chlore ou de brome, avec une amine secondaire de formule générale V :

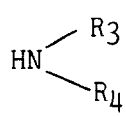

(V)

dans laquelle $R_3$ et $R_4$ ont les significations définies dans la revendication 1.

**14.** Les compositions pharmaceutiques contenant comme principe actif un dérivé selon les revendications 1 et 3 à 11, avec des excipients pharmaceutiques appropriés.

**15.** Les compositions pharmaceutiques selon la revendication 14 présentées sous une forme convenant notamment pour le traitement des asthénies, des syndromes ischémiques aigus transitoires ou progressifs et des maladies nerveuses liées au vieillissement normal ou pathologique.

**Revendications pour les Etats contractants suivants : ES, GR**

**1.** Le procédé de préparation des dérivés de la pyrrolidone de formule générale I ;

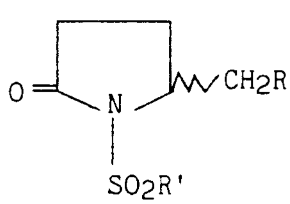

(I)

dans laquelle :

- R représente :
  un groupe

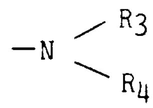

dans lequel :

. $R_3$ et $R_4$, identiques ou différents, représentent chacun : un atome d'hydrogène, un radical alkyle ayant jusqu'à 6 atomes de carbone, en chaîne droite ou ramifiée, ou

. $R_3$ et $R_4$ forment ensemble avec l'atome d'azote auquel ils sont liés un radical hétérocyclique de formule

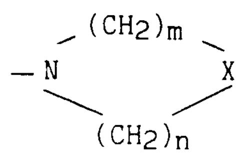

dans laquelle :

- m représente zéro, un ou deux ;
- n est un nombre entier de 1 à 3, et
- X représente :
- un radical méthylène - $CH_2$ - ;
- un atome d'oxygène ou de soufre, ou le radical $SO_2$ ;
- un radical N-$R_5$ dans lequel $R_5$ représente :
  . un radical alkyle ayant de 1 à 4 atomes de carbone,
  . un radical hydroxyalkyle ou aralkyle dans lequel la partie aryle est éventuellement substituée par un ou plusieurs radicaux alkoxy (ayant de 1 à 4 atomes de carbone) ou méthylènedioxy, et où, dans chacun d'entre eux, la partie alkyle renferme de 1 à 4 atomes de carbone,
  . un radical aryle éventuellement substitué par un ou plusieurs atomes d'halogène ou radicaux alkoxy ayant de 1 à 4 atomes de carbone, ou
  . un radical hétérocyclique, ayant un ou deux hétéroatomes, choisi parmi les radicaux thiazolyle, pyrimidinyle, pyrazinyle pyridyle et quinolyle, et chacun de ces radicaux substitué par un ou plusieurs atomes d'halogène ou radicaux trifluorométhyle ou alkoxy ayant de 1 à 4 atomes de carbone ; ou,
- un radical

  $>$CH - YZ

  dans lequel Y représente un atome d'oxygène ou de soufre et Z représente un radical hétérocyclique, ayant 1 ou 2 hétéroatomes, tel que défini ci-dessus ;

- R' représente :
  a) un radical alkyle ayant de 1 à 4 atomes de carbone en chaîne droite ou ramifiée, comportant éventuellement une double liaison et / ou éventuellement substitué par un ou plusieurs radicaux hydroxy, trifluorométhyle, amino ou phényle lui-même éventuellement substitué par un ou plusieurs atomes d'halogène ou radicaux alkyle ou alkoxy (ayant chacun de 1 à 5 atomes de carbone), nitro ou amino ; ou
  b) un radical aryle éventuellement substitué par un ou plusieurs atomes d'halogène ou radicaux alkyle ou alkoxy (ayant chacun de 1 à 5 atomes de carbone), nitro ou amino,
  et,
- le carbone situé en position 5 du cycle pyrrolidone, substitué par $CH_2R$, possède la configuration R ou S

et leurs sels d'addition avec des acides appropriés,
caractérisé en ce que l'on fait réagir un dérivé de la pyrrolidone de formule générale II :

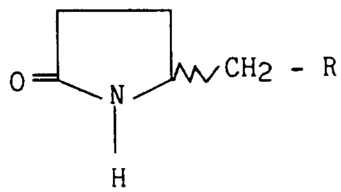

(II)

dans laquelle R a la signification précédemmment définie, avec un sulfochlorure de formule générale III :

Cl $SO_2$ - R' (III)

dans laquelle R' a la signification précédemment définie et, si on le désire, on traite les dérivés ainsi obtenus avec des acides appropriés, pour donner les sels d'addition correspondants.

**2.** Le procédé de préparation des dérivés de formule I, définie dans la revendication 1 et leurs sels d'addition avec des acides appropriés, caractérisé en ce que l'on fait réagir un dérivé de la pyrrolidone de formule générale IV :

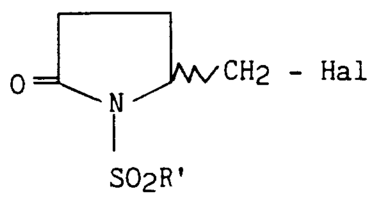

(IV)

dans laquelle R' a la signification définie dans la revendication 1 et Hal représente un atome de chlore ou de brome, avec une amine secondaire de formule générale V :

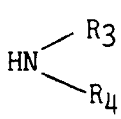

(V)

dans laquelle $R_3$ et $R_4$ ont les significations définies dans la revendication 1 ; et, si on le désire, on traite les dérivés ainsi obtenus avec des acides appropriés, pour donner les sels d'addition correspondants.

**Claims**

**Claims for the following Contracting States : AT, BE, CH, DE, DK, FR, GB, IT, LI, LU, NL, SE**

**1.** Pyrrolidone compounds of the general formula I:

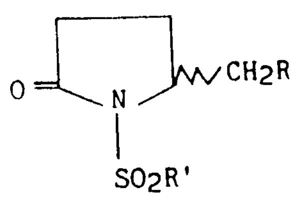

(I)

in which:

- R represents:

  a group

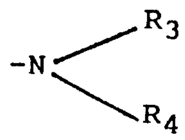

in which:

. $R_3$ and $R_4$, which are identical or different, each represent:
a hydrogen atom, or
a straight-chain or branched alkyl radical having up to 6 carbon atoms, or

. $R_3$ and $R_4$, together with the nitrogen atom to which they are bonded, form a heterocyclic radical of the formula

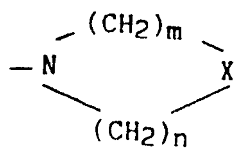

in which:

- m represents zero, one or two;
- n is an integer of from 1 to 3 and
- X represents:
  - a methylene radical $-CH_2-$;
  - an oxygen or sulphur atom, or the radical $SO_2$;
  - a radical N - $R_5$ in which $R_5$ represents:
    . an alkyl radical having from 1 to 4 carbon atoms,
    . a hydroxyalkyl or aralkyl radical in which the aryl moiety is optionally substituted by one or more alkoxy radicals (having from 1 to 4 carbon atoms) or methylenedioxy radicals, and in each of which the alkyl moiety contains from 1 to 4 carbon atoms,
    . an aryl radical that is optionally substituted by one or more halogen atoms or alkoxy radicals having from 1 to 4 carbon atoms, or
    . a heterocyclic radical having one or two hetero atoms, selected from the radicals thiazolyl, pyrimidinyl, pyrazinyl, pyridyl and quinolyl, each of those radicals being substituted by one or more halogen atoms, trifluoromethyl radicals or alkoxy radicals having from 1 to 4 carbon atoms; or
  - a radical

    $>$CH - YZ

    in which Y represents an oxygen or sulphur atom and Z represents a heterocyclic radical having one or two hetero atoms, as defined above;

- R' represents:
a) a straight-chain or branched alkyl radical having from 1 to 4 carbon atoms that optionally contains a double bond and/or is optionally substituted by one or more of the following radicals: hydroxy, trifluoromethyl, amino, or phenyl that is itself optionally substituted by one or more halogen atoms or alkyl or alkoxy radicals (each having from 1 to 5 carbon atoms), nitro or amino radicals; or
b) an aryl radical that is optionally substituted by one or more halogen atoms or alkyl or alkoxy radicals (each having from 1 to 5 carbon atoms), nitro or amino radicals;
and
- the carbon located in the 5-position of the pyrrolidine ring, which is substituted by $CH_2R$, has the R or S configuration.

**2.** The acid addition salts of the compounds of claim 1.

**3.** The salts according to claim 2 that are physiologically tolerable.

4. A compound according to claim 1, which is: S-1-benzenesulphonyl-2-oxo-5-morpholinomethyl-pyrrolidine.

5. A compound according to claim 1, which is: S-1-benzenesulphonyl-2-oxo-5-[(4-methyl-1-piperazinyl)-methyl]-pyrrolidine.

6. A compound according to claim 1, which is: S-1-benzenesulphonyl-2-oxo-5-[(4-metatrifluoromethylphenyl-1-piperazinyl)methyl]-pyrrolidine.

7. A compound according to claim 1, which is: S-1-methanesulphonyl-2-oxo-5-morpholinomethyl-pyrrolidine.

8. A compound according to claim 1, which is: S-1-benzenesulphonyl-2-oxo-5-{[4-(6-chloro-2-pyrazinyl)-1-piperazinyl]methyl}-pyrrolidine, and its hydrochloride.

9. A compound according to claim 1, which is: S-1-benzenesulphonyl-2-oxo-5-morpholinomethyl-pyrrolidine, and its hydrochloride.

10. A compound according to claim 1, which is: S-1-styrylsulphonyl-2-oxo-5-[(4-methyl-1-piperazinyl)-methyl]-pyrrolidine.

11. A compound according to claim 1, which is: S-1-styrysulphonyl-2-oxo-5-thiomorpholinomethyl-pyrrolidine.

12. A process for the preparation of the compounds of claim 1, characterised in that a pyrrolidone compound of the general formula II:

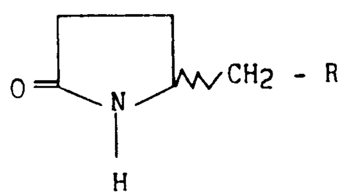

(II),

in which R has the meaning defined in claim 1, is reacted with a sulphonyl chloride of the general formula III:

$ClSO_2$ - R'     (III)

in which R' has the meaning defined in claim 1.

13. A process for the preparation of the compounds of claim 1, characterised in that a pyrrolidone compound of the general formula IV:

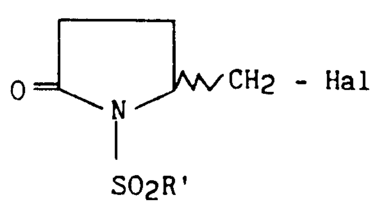

(IV),

in which R' has the meaning defined in claim 1 and Hal represents a chlorine or bromine atom, is reacted with a secondary amine of the general formula V:

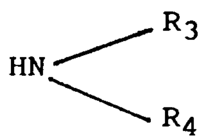

(V)

in which $R_3$ and $R_4$ have the meanings defined in claim 1.

**14.** Pharmaceutical compositions containing as active ingredient a compound according to claims 1 and 3 to 11, with suitable pharmaceutical excipients.

**15.** Pharmaceutical compositions according to claim 14 in a form suitable in particular for the treatment of asthenias, acute transitory or progressive ischaemic syndromes and nervous disorders associated with normal or pathological ageing.

**Claims for the following Contracting States : ES, GR**

**1.** A process for the preparation of pyrrolidone compounds of the general formula I:

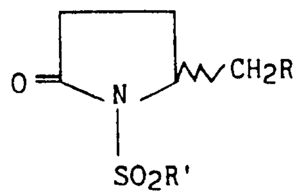

(I)

in which:

- R represents:
  a group

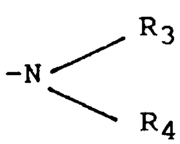

in which:

. $R_3$ and $R_4$, which are identical or different, each represent:
a hydrogen atom, or
a straight-chain or branched alkyl radical having up to 6 carbon atoms, or

. $R_3$ and $R_4$, together with the nitrogen atom to which they are bonded, form a heterocyclic radical of the formula

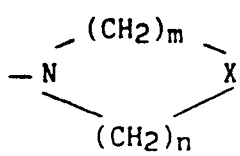

in which:

- m represents zero, one or two;
- n is an integer of from 1 to 3 and
- X represents:
  - a methylene radical $-CH_2-$;
  - an oxygen or sulphur atom, or the radical $SO_2$;

- a radical N - $R_5$ in which $R_5$ represents:
  . an alkyl radical having from 1 to 4 carbon atoms,
  . a hydroxyalkyl or aralkyl radical in which the aryl moiety is optionally substituted by one or more alkoxy radicals (having from 1 to 4 carbon atoms) or methylenedioxy radicals, and in each of which the alkyl moiety contains from 1 to 4 carbon atoms,
  . an aryl radical that is optionally substituted by one or more halogen atoms or alkoxy radicals having from 1 to 4 carbon atoms, or
  . a heterocyclic radical having one or two hetero atoms, selected from the radicals thiazolyl, pyrimidinyl, pyrazinyl, pyridyl and quinolyl, each of those radicals being substituted by one or more halogen atoms, trifluoromethyl radicals or alkoxy radicals having from 1 to 4 carbon atoms; or
- a radical

  $>$CH - YZ

  in which Y represents an oxygen or sulphur atom and Z represents a heterocyclic radical having one or two hetero atoms, as defined above;
- R' represents:
  a) a straight-chain or branched alkyl radical having from 1 to 4 carbon atoms that optionally contains a double bond and/or is optionally substituted by one or more of the following radicals: hydroxy, trifluoromethyl, amino, or phenyl that is itself optionally substituted by one or more halogen atoms or alkyl or alkoxy radicals (each having from 1 to 5 carbon atoms), nitro or amino radicals; or
  b) an aryl radical that is optionally substituted by one or more halogen atoms or alkyl or alkoxy radicals (each having from 1 to 5 carbon atoms), nitro or amino radicals;
  and
- the carbon located in the 5-position of the pyrrolidine ring, which is substituted by $CH_2R$, has the R or S configuration,

and the addition salts thereof with appropriate acids,
characterised in that a pyrrolidone compound of the general formula II:

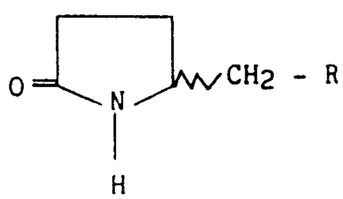

(II),

in which R has the meaning defined above, is reacted with a sulphonyl chloride of the general formula III:

$ClSO_2$ - R'     (III)

in which R' has the meaning defined above, and, if desired, the compounds so obtained are treated with appropriate acids to yield the corresponding addition salts.

**2.** A process for the preparation of the compounds of formula I defined in claim 1, and the addition salts thereof with appropriate acids, characterised in that a pyrrolidone compound of the general formula IV:

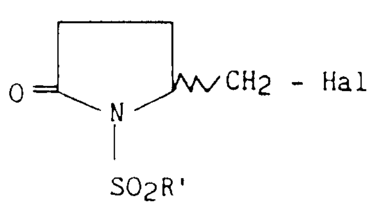

(IV),

in which R' has the meaning defined in claim 1 and Hal represents a chlorine or bromine atom, is reacted with a secondary amine of the general formula V:

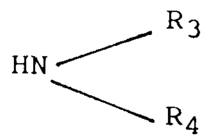

(V)

in which $R_3$ and $R_4$ have the meanings defined in claim 1,
and, if desired, the compounds so obtained are treated with appropriate acids to yield the corresponding addition salts.

**Patentansprüche**

**Patentansprüche für folgende Vertragsstaaten : AT, BE, CH, DE, DK, FR, GB, IT, LI, LU, NL, SE**

**1.** Pyrrolidonderivate der allgemeinen Formel I:

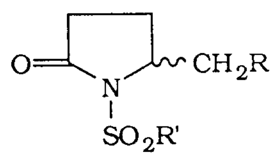

(I)

in der

- R:

eine Gruppe

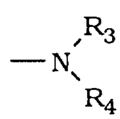

in der:

. $R_3$ und $R_4$, die gleichartig oder verschieden sein können, jeweils ein Wasserstoffatom, eine Alkylgruppe mit bis zu 6 Kohlenstoffatomen in gerader oder verzweigter Kette oder

. $R_3$ und $R_4$ gemeinsam mit dem Stickstoffatom, an das sie gebunden sind, einen heterocyclischen Rest der Formel

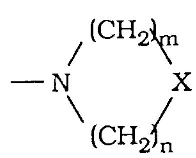

in der:

- m Null, Eins oder Zwei;
- n eine ganze Zahl mit einem Wert von 1 bis 3; und
- X:
  - eine Methylengruppe $-CH_2-$;
  - ein Sauerstoffatom oder ein Schwefelatom oder eine Gruppe $SO_2$;
  - eine Gruppe $N-R_5$, In der $R_5$:
    . eine Alkylgruppe mit 1 bis 4 Kohlenstoffatomen,

. eine Hydroxyalkyl- oder Aralkylgruppe, in der der Arylrest gegebenenfalls durch eine oder mehrere Alkoxygruppen (mit 1 bis 4 Kohlenstoffatomen) oder Methylendioxygruppen substituiert ist und worin in beiden Fällen der Alkylrest 1 bis 4 Kohlenstoffatome umfaßt,

. eine Arylgruppe, die gegebenenfalls durch eine oder mehrere Halogenatome oder Alkoxygruppen mit 1 bis 4 Kohlenstoffatomen substituiert ist, oder

. eine heterocyclische Gruppe mit einem oder zwei Heteroatomen ausgewählt aus Thiazolyl-, Pyrimidinyl-, Pyrazinyl-, Pyridyl- oder Chinolyl-resten, wobei jeder dieser Reste durch eines oder mehrere Halogenatome oder Trifluormethylgruppen oder Alkoxygruppen mit 1 bis 4 Kohlenstoffatomen substituiert ist, darstellt; oder

- eine Gruppe

  $>CH\text{-}YZ$

  in der Y ein Sauerstoffatom oder ein Schwefelatom und Z einen heterocyclischen Rest mit 1 oder 2 Heteroatomen, wie er oben definiert worden ist, bedeuten; darstellen und

- R':

  a) eine Alkylgruppe mit 1 bis 4 Kohlenstoffatomen in gerader oder verzweigter Kette, die gegebenenfalls eine Doppelbindung aufweist und/oder gegebenenfalls substituiert ist durch eine oder mehrere Hydroxylgruppen, Trifluormethylgruppen, Aminogruppen oder Phenylgruppen, die ihrerseits gegebenenfalls durch eines oder mehrere Halogenatome oder Alkyl- oder Alkoxygruppen (die jeweils 1 bis 5 Kohlenstoffatome aufweisen), Nitro- oder Aminogruppen substituiert sind; oder

  b) eine Arylgruppe, die gegebenenfalls durch eines oder mehrere Halogenatome oder Alkyl- oder Alkoxygruppen (die jeweils 1 bis 5 Kohlenstoffatome umfassen), Nitro- oder Aminogruppen substituiert ist, bedeuten

  und

- das Kohlenstoffatom in der 5-Stellung des Pyrrolidinrings, das durch $CH_2R$ substituiert ist, die Konfiguration R oder S besitzt.

**2.** Säureadditionssalze der Derivate nach Anspruch 1

**3.** Salze nach Anspruch 2, die physiologisch verträglich sind.

**4.** Verbindung nach Anspruch 1, nämlich S-1-Benzolsulfonyl-2-oxo-5-morpholinomethyl-pyrrolidin.

**5.** Verbindung nach Anspruch 1, nämlich S-1-Benzolsulfonyl-2-oxo-5-[(4-methyl-piperazin-1-yl)-methyl]-pyrrolidin.

**6.** Verbindung nach Anspruch 1, nämlich S-1-Benzolsulfonyl-2-oxo-5-[(4-m-trifluormethylphenyl-piperazin-1-yl)-methyl]-pyrrolidin.

**7.** Verbindung nach Anspruch 1, nämlich S-1 Methansulfonyl-2-oxo-5-morpholinomethyl-pyrrolidin.

**8.** Verbindung nach Anspruch 1, nämlich S-1-Benzolsulfonyl-2-oxo-5-{[4-(6-chlor-pyrazin-2-yl)-piperazin-1-yl]-methyl}-pyrrolidin und dessen Hydrochlorid.

**9.** Verbindung nach Anspruch 1, nämlich S-1-Benzylsulfonyl-2-oxo-5-morpholinomethyl-pyrrolidin und dessen Hydrochlorid.

**10.** Verbindung nach Anspruch 1, nämlich S-1-Styrylsulfonyl-2-oxo-5-[(4-methyl-piperazin-1-yl)-methyl]-pyrrolidin.

**11.** Verbindung nach Anspruch 1, nämlich S-1 Styrylsulfonyl-2-oxo-5-thiomorpholinomethyl-pyrrolidin.

**12.** Verfahren zur Herstellung der Derivate nach Anspruch 1, **dadurch gekennzeichnet**, daß man ein Pyrrolidonderivat der allgemeinen Formel II:

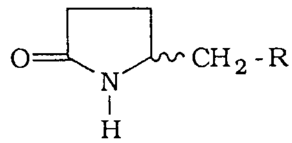

(II)

in der R die in Anspruch 1 angegebenen Bedeutungen besitzt, mit einem Sulfochlorid der allgemeinen Formel III:

$Cl\ SO_2$-R' (III)

in der R' die in Anspruch 1 angegebenen Bedeutungen besitzt, umsetzt.

13. Verfahren zur Herstellung der Derivate nach Anspruch 1, **dadurch gekennzeichnet**, daß man ein Pyrrolidonderivat der allgemeinen Formel IV:

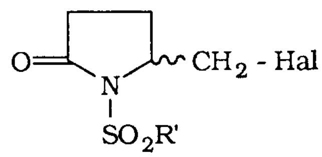

(IV)

in der R' die in Anspruch 1 angegebenen Bedeutungen besitzt und Hal ein Chlor- oder Bromatom darstellt, mit einem sekundären Amin der allgemeinen Formel V:

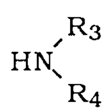

(V)

in der $R_3$ und $R_4$ die in Anspruch 1 angegebenen Bedeutungen besitzen, umsetzt.

14. Pharmazeutische Zubereitungen enthaltend als Wirkstoff ein Derivat nach einem der Ansprüche 1 und 3 bis 11 und pharmazeutisch geeignete Trägermaterialien.

15. Pharmazeutische Zubereitungen nach Anspruch 14 in einer Form, die insbesondere zur Behandlung von Asthenien, akuten, vorübergehenden oder progressiven ischämischen Syndromen und nervösen Erkrankungen, die mit dem normalen oder pathologischen Altern verknüpft sind, geeignet ist, vorliegen.

**Patentansprüche für folgende Vertragsstaaten : ES, GR**

1. Verfahren zur Herstellung der Pyrrolidonderivate der allgemeinen Formel I:

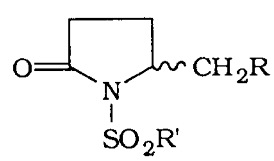

(I)

in der

- R:

  eine Gruppe

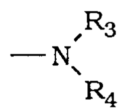

in der:

. $R_3$ und $R_4$, die gleichartig oder verschieden sein können, jeweils ein Wasserstoffatom, eine Alkylgruppe mit bis zu 6 Kohlenstoffatomen in gerader oder verzweigter Kette oder

. $R_3$ und $R_4$ gemeinsam mit dem Stickstoffatom, an das sie gebunden sind, einen heterocyclischen Rest der Formel

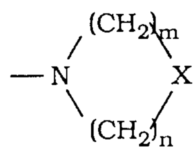

in der:

- m Null, Eins oder Zwei;
- n eine ganze Zahl mit einem Wert von 1 bis 3; und
- X:
  - eine Methylengruppe $-CH_2-$;
  - ein Sauerstoffatom oder ein Schwefelatom oder eine Gruppe $So_2$;
  - eine Gruppe $N-R_5$, in der $R_5$:
    . eine Alkylgruppe mit 1 bis 4 Kohlenstoffatomen,
    . eine Hydroxyalkyl- oder Aralkylgruppe, in der der Arylrest gegebenenfalls durch eine oder mehrere Alkoxygruppen (mit 1 bis 4 Kohlenstoffatomen) oder Methylendioxygruppen substituiert ist und worin in beiden Fällen der Alkylrest 1 bis 4 Kohlenstoffatome umfaßt,
    . eine Arylgruppe, die gegebenenfalls durch eine oder mehrere Halogenatome oder Alkoxygruppen mit 1 bis 4 Kohlenstoffatomen substituiert ist, oder
    . eine heterocyclische Gruppe mit einem oder zwei Heteroatomen ausgewählt aus Thiazolyl-, Pyrimidinyl-, Pyrazinyl-, Pyridyl- oder Chinolyl-resten, wobei jeder dieser Reste durch eines oder mehrere Halogenatome oder Trifluormethylgruppen oder Alkoxygruppen mit 1 bis 4 Kohlenstoffatomen substituiert ist, darstellt; oder
- eine Gruppe

  >CH-YZ

  in der Y ein Sauerstoffatom oder ein Schwefelatom und Z einen heterocyclischen Rest mit 1 oder 2 Heteroatomen, wie er oben definiert worden ist, bedeuten; darstellen und

- R':

  a) eine Alkylgruppe mit 1 bis 4 Kohlenstoffatomen in gerader oder verzweigter Kette, die gegebenenfalls eine Doppelbindung aufweist und/oder gegebenenfalls substituiert ist durch eine oder mehrere Hydroxylgruppen, Trifluormethylgruppen, Aminogruppen oder Phenylgruppen, die ihrerseits gegebenenfalls durch eines oder mehrere Halogenatome oder Alkyl- oder Alkoxygruppen (die jeweils 1 bis 5 Kohlenstoffatome aufweisen), Nitro- oder Aminogruppen substituiert sind; oder

  b) eine Arylgruppe, die gegebenenfalls durch eines oder mehrere Halogenatome oder Alkyl- oder Alkoxygruppen (die jeweils 1 bis 5 Kohlenstoffatome umfassen), Nitro- oder Aminogruppen substituiert ist, bedeuten

  und

- das Kohlenstoffatom in der 5-Stellung des Pyrrolidinrings, das durch $CH_2R$ substituiert ist, die Konfiguration R oder S besitzt,

und von deren Additionssalzen mit geeigneten Säuren,

**dadurch gekennzeichnet**, daß man ein Pyrrolidonderivat der allgemeinen Formel II:

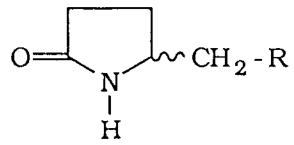

(II)

in der R die oben angegebenen Bedeutungen besitzt, mit einem Sulfochlorid der allgemeinen Formel III:

Cl $SO_2$-R' (III)

in der R' die oben angegebenen Bedeutungen besitzt, umsetzt und gewünschtenfalls die in dieser Weise erhaltenen Derivate zur Bildung der entsprechenden Additionssalze mit geeigneten Säuren behandelt.

**2.** Verfahren nach Anspruch 1 zur Herstellung der Derivate der Formel I und von deren Additionssalzen mit geeigneten Säuren, **dadurch gekennzeichnet**, daß man ein Pyrrolidonderivat der allgemeinen Formel IV:

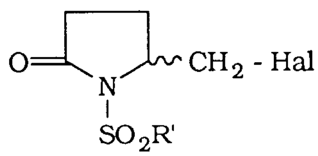

(IV)

in der R' die in Anspruch 1 angegebenen Bedeutungen besitzt und Hal ein Chlor- oder Bromatom darstellt, mit einem sekundären Amin der allgemeinen Formel V:

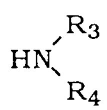

(V)

in der $R_3$ und $R_4$ die in Anspruch 1 angegebenen Bedeutungen besitzen, umsetzt; und gewünschtenfalls die in dieser Weise erhaltenen Derivate zur Bildung der entsprechenden Additionssalze mit geeigneten Säuren behandelt.